# EUROPEAN PATENT SPECIFICATION

(11) **EP 2 080 487 B1**
(45) Date of publication and mention of the grant of the patent: **30.06.2010**
(21) Application number: 08425021.6
(22) Date of filing: 16.01.2008
(51) Int. Cl.: A61B 19/00, A61M 25/09, A61B 17/00, A61B 17/34

(54) **Guide device for localising a neoplasia to be removed during a surgical procedure**
Führungsvorrichtung zur Lokalisierung von Neoplasien während eines chirurgischen Verfahrens
Dispositif de guidage pour localiser une néoplasie à extraire lors d'une intervention chirurgicale

(43) Date of publication of application: 22.07.2009
(73) Proprietor: Zambelli, Roberto, 20017 Rho (MI) (IT)
(72) Inventor: Zambelli, Roberto, 20017 Rho (MI) (IT)
(74) Representative: Petruzziello, Aldo

(56) References cited:
- EP-A- 0 481 685
- WO-A-00/24332
- WO-A-96/10953
- US-A- 5 989 265
- US-A- 6 056 700
- US-A1- 2003 195 433

## Description

The present invention pertains to a guide device to localise a neoplasia that is to be removed during a surgical procedure. A guide device as defined in the preamble of claim 1 is disclosed in EP 0 481 685.

When, during an ultrasound or radiological examination on a patient, in particular a mammography, a neoplasia such as a nodule requiring to be removed by a surgical procedure is discovered, the surgeon must be allowed to identify and to localise the neoplasia to be removed with the greatest precision possible. This means that a guide system capable of guiding the surgical instruments exactly to the target to be removed must be provided.

Guide devices are known on the market that comprise a needle cannula within which a metal wire, commonly known as a guide wire is slidably disposed. The needle cannula has a percutaneous tip adapted to penetrate the patient's body, generally a woman's breast, until it reaches the area in which the neoplasia to be removed is situated. The guide wire is then advanced inside the needle cannula or the needle cannula is retracted with respect to the guide wire, so that the tip of the guide wire exits from the tip of the needle cannula to anchor itself near the neoplasia to be removed. Lastly, the needle cannula is extracted from the patient's body, leaving the guide wire anchored near the neoplasia to act as a guide for the surgeon.

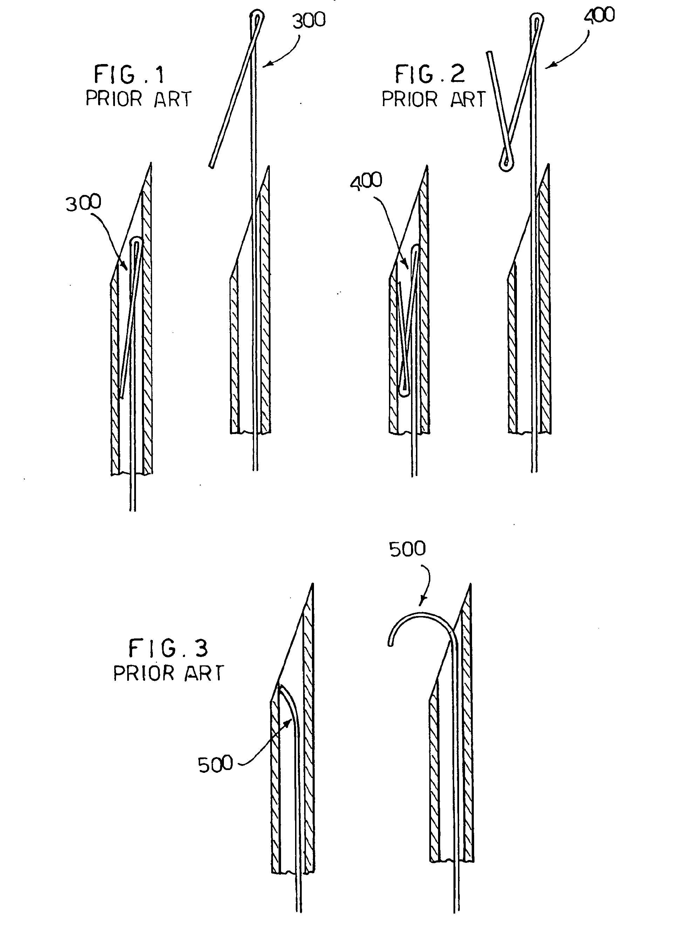

As shown in Figure 1, guide devices are known to the art, which have a guide wire with a V-shaped tip 300, which anchors itself easily in the area of the neoplasia. This type of guide device has the drawback that the guide wire tends to migrate from the anchoring site. In fact, the V-shaped shape of the tip prevents the shifting of the guide wire in the direction of removal (backwards), but it allows the shifting thereof in the direction of insertion (forwards).

As shown in Figure 2, to overcome at least in part this drawback of the migration, guide wires have been devised with a double V-shaped tip 400. Such types of guide wires remain locked in position since they cannot move forwards or backwards.

However, during radiological or ultrasound examination, it often happens that the tip of the guide wire has not been positioned correctly near the neoplasia and, therefore, it must be moved and repositioned near the neoplasia. Guide wires with a V-shaped or double V-shaped tip cannot be repositioned. In fact, once the tip of the guide wire has been extracted from the tip of the needle cannula, it is impossible to make it return inside the needle cannula again since the crooks of the V or double V would jam in the edge of the tip of the needle cannula.

To overcome said drawback, repositionable guide wires are known on the market made of a memory-shape metal wire, that is of a metal wire which, after being deformed, resumes exactly its original shape. As shown in Figure 3, the repositionable guide wire has a J-shaped tip 500. When the tip of the guide wire is disposed inside the needle cannula, it is deformed taking on a substantially straight shape. When the tip of the guide wire is extracted from the needle cannula, it resumes its original J-shape, anchoring itself to the body tissue.

In this manner the tip of the guide wire can be extracted from the needle cannula and retracted inside the needle cannula a number of times, making the guide wire repositionable.

However, the repositionable guide wires have the drawback that they tend to migrate, since the J-shaped tip wraps around itself and tends to rotate with respect to the anchoring site.

Furthermore, the guide wire must be sufficiently stiff to be able to be guided inside the needle cannula. This excessive stiffness of the guide wire leads to various drawbacks. In fact, it must be considered that the guide wire remains implanted in the patient's body for as long as a few days before the surgical procedure for removal of the neoplasia takes place. Therefore, an excessively stiff guide wire proves inconvenient and uncomfortable for the patient.

Furthermore, it must be considered that the patient does not remain perfectly immobile. Thus an excessively stiff guide wire tends to transmit to its tip tensions and stresses due to movement of the patient's body. As a result, the tip of the guide wire tends to migrate from the anchoring site.

Other types of guide devices are known, which, however, are particularly complex and have excessively stiff guide wires, which are thus particularly inconvenient and uncomfortable for the patient.

The guide devices known to the art must generally be operated with both hands. In fact the operator holds a support, which supports the needle cannula, with one hand and holds another support, which supports the guide, wire with the other hand. This type of two-handed operation is possible only when an X-ray apparatus which has a special positioning seat for the guide device is used. Thus the operator has both hands free to operate the guide device.

However, it must be considered that, in the case of use of an ultrasound apparatus, the operator has one hand occupied in holding the ultrasound probe. Therefore the need arises to have to use the guide device with only one hand. In this case the guide device has a three-ringed grip in which the central ring is made integral with the needle cannula and the two side rings, on the other hand, are constrained to the guide wire. In this manner the guide device can be operated only with three fingers of one hand.

Object of the present invention is to overcome the drawbacks of the prior art, by providing a guide device to localise a neoplasia that must be removed during a surgical procedure, that can be repositioned and that, at the same time, is not liable to migrate inside the patient's body.

Another object of the present invention is to provide such a guide device that is simple for the operator to use and at the same time is convenient and comfortable for the patient.

These objects are achieved in accordance with the invention with the characteristics listed in the appended independent claim 1.
Advantageous embodiments of the invention are apparent from the dependent claims.

According to the invention, the guide device to localise a neoplasia which must be removed during a surgical procedure comprises:
- a support, which supports a cannula, hollow on the inside, provided with a percutaneous tip adapted to penetrate the patient's body, and
- a guide wire assembly adapted to be inserted slidably in the cannula and provided with a tip adapted to anchor itself to a body tissue inside the patient's body.

The tip of the guide wire is made from a memory-shape metal wire that is initially shaped as a curl that forms at least a 360° loop. The use of the memory-shape metal wire and its particular curled shape allow a firm anchoring of the tip of the guide wire to the patient's body tissues. The tip of the guide wire can be easily inserted and extracted a number of times from the needle cannula without losing its initial configuration, thus making the guide device repositionable.

Further characteristics of the invention will be made clearer by the detailed description that follows, referring to purely exemplifying and therefore non limiting embodiments thereof, illustrated in the appended drawings, wherein:
Figures 1, 2 e 3 are axial sectional views, enlarged and partially broken off, illustrating respectively three different tips of guide wires according to the prior art in a retracted position inside the percutaneous tip of the needle cannula and in an extracted position outside the percutaneous tip of the needle cannula;
Figure 4 is a plan view illustrating exploded a first embodiment of the guide device according to the invention;
Figure 5 is an enlarged axial sectional view, illustrating the distal part of the guide device according to the invention, in which the tip of the guide wire is retracted inside the needle cannula;
Figure 6 is a view like Figure 5, but illustrating the tip of the guide wire extracted from the needle cannula;
Figure 7 is a top plan view, illustrating exploded a slider of the cannula and a slider of the stylet according to a second embodiment of the guide device according to the invention;
Figure 8 is a top plan view illustrating the guide device of Figure 7 assembled;
Figure 9 is a bottom plan view of the slider of the cannula of Figure 7;
Figure 10 is a side view, partially sectioned, of the slider of the cannula of Figure 7;
Figure 11 is an enlarged cross sectional view taken along the plane of section XI-XI of
Figure 10;

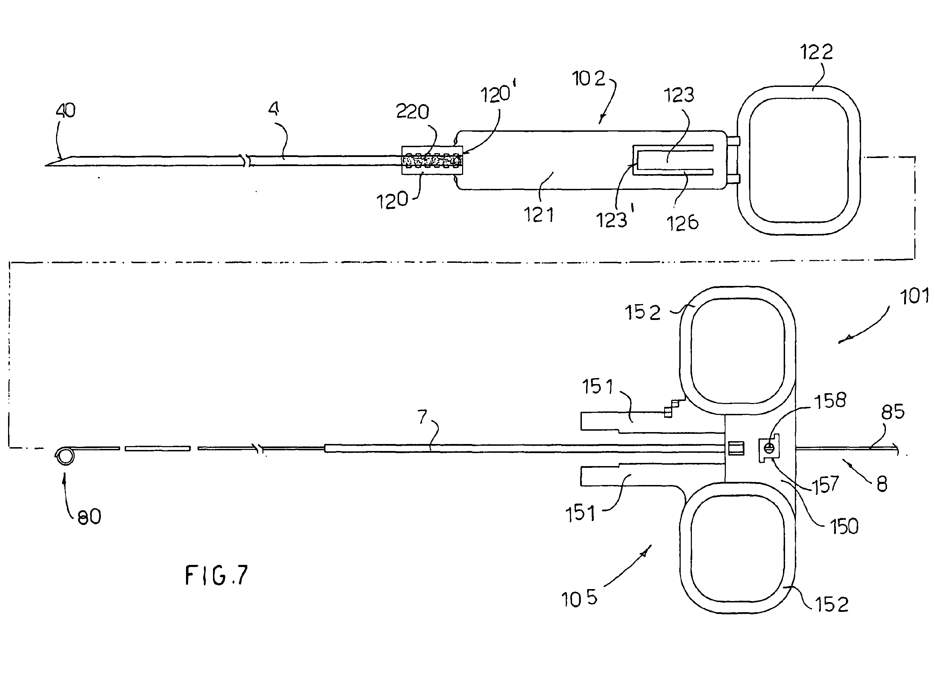

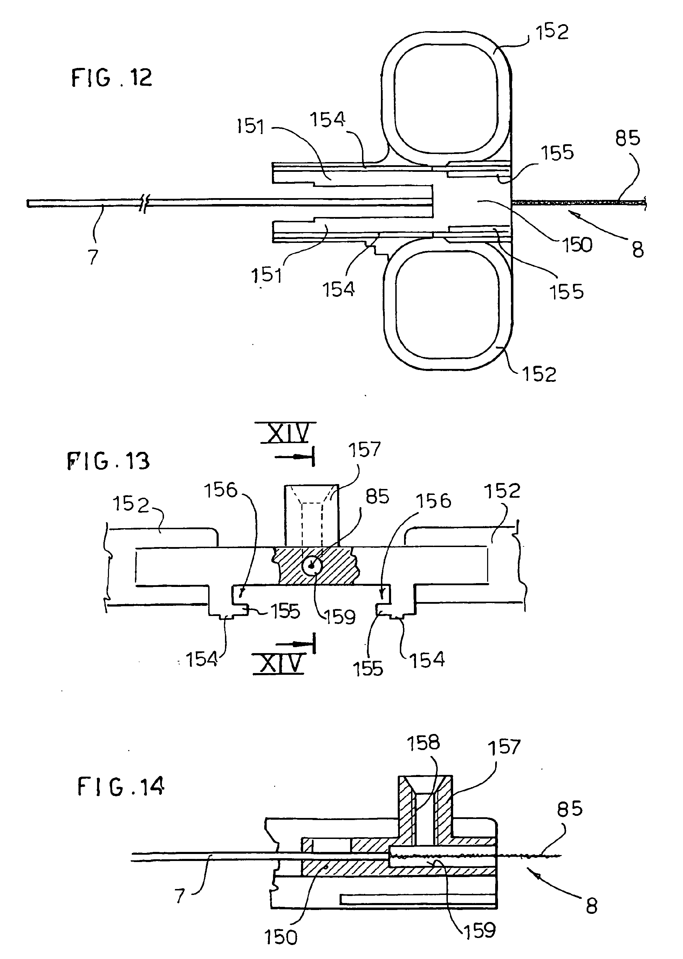

Figure 12 is a bottom plan view of the slider of the stylet of Figure 7;
Figure 13 is an enlarged rear view, partially sectioned and partially broken off, of the slider of the stylet of Figure 7; and
Figure 14 is an axial sectional view, enlarged and broken off, taken along the plane of section XIV-XIV of Figure 13.

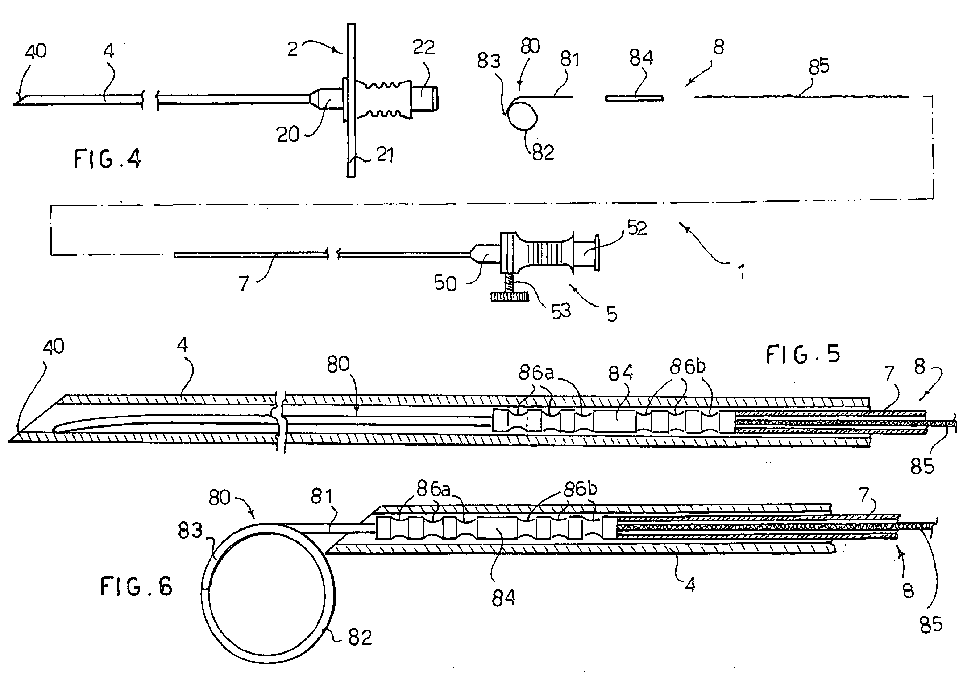

With reference for now to Figures 4-6, a first embodiment of the guide device according to the invention, designated as a whole with the reference numeral 1, is described.

The guide device 1 comprises:
- a first support 2, which supports a cannula 4, hollow on the inside, provided with a percutaneous tip 40 adapted to penetrate the patient's body;
- a second support 5 which supports a stylet 7, hollow on the inside, adapted to be inserted slidably in the cannula 4, and
- a guide wire assembly 8 adapted to be inserted slidably in the stylet 7 and provided with a tip 80 adapted to anchor itself within the patient's body.

Taking the operator as a reference, in the following the term "proximal" indicates the parts of the device near the operator, whereas the term "distal" indicates the parts of the device away from the operator.

The cannula support 2 comprises a cylindrical or truncated conical distal tang 20 in which the cannula 4 is fixed axially and a proximal tang 22, hollow on the inside and open at its proximal end to allow the insertion of the stylet 7 inside the cannula 4. The cannula 4 consists of a metal tube hollow on the inside with a percutaneous tip 40 and an outside diameter of about 1 mm. Between the distal tang 20 and the proximal tang 22 there is an intermediate flange 21 that protrudes radially therefrom and offers a rest for the user's fingers.

The stylet support 5 comprises a cylindrical or truncated conical distal tang 50 in which the stylet 7 is fixed axially. The stylet 7 consists of a rigid cylindrical tube, hollow on the inside, having an outside diameter slightly smaller than the inside diameter of the cannula 4. The distal tang 50 of the stylet support 5 is adapted to be inserted in the proximal tang 22 of the cannula support 2.

The stylet support 5 comprises a proximal tang 52, hollow on the inside, communicating with the inside of the stylet 7 and open at its proximal end to allow the guide wire assembly 8 to exit. A screw 53 adapted to lock the guide wire assembly 8 in position integrally with the stylet support 5 screws radially in an intermediate position in the body of the stylet support 5.

The guide wire assembly 8 comprises a tip 80 made with a memory-shape metal wire having a diameter of about 0.3-0.6 mm. The tip 80 is preferably made with a nickel-titanium alloy wire, known by the commercial name NiTiNol, which consists of 55% nickel and 45% titanium.

The tip 80 is given the initial shape of a curl; that is, it is wound on itself by one turn plus a quarter of a turn. Precisely, the tip 80 has:
- a straight proximal part 81 that extends about 3-6 mm,
- an intermediate part 82 which forms a loop of 360°, that is a circumference with a diameter of about 5-10 mm, preferably 8 mm,
- a distal part 83 which forms an arc of about 30°-60°, preferably 45°, around the intermediate part 82.

The proximal part 81 of the tip of the guide wire 8 is fixed to one end of a substantially rigid connecting sleeve 84, of biocompatible metal material such as stainless steel. The connecting sleeve is formed by a capillary tube having an outside diameter larger than the inside diameter of the stylet 7 and smaller than the inside diameter of the cannula 4. The other end of the connecting sleeve 84 is secured to a braid of metal wires 85, of biocompatible metal such as stainless steel, with an extremely small diameter (a few microns), so as to be not very stiff and very flexible. The outside diameter of the braid 85 is smaller than the outside diameter of the memory-shape wire of the tip 80 and the stiffness of the braid 85 is less than the stiffness of the wire of the tip 80.

In any case, both the outside diameter of the memory-shape wire of the tip 80 and the outside diameter of the braid 85 are smaller than the inside diameter of the connecting sleeve 84. The distal end 81 of the tip 80 and the proximal end of the braid 85 are then inserted into the connecting sleeve 84 and secured therein by crimping 86a and 86b (Figures 5 and 6). Furthermore, the outside diameter of the braid 85 must be smaller than the inside diameter of the stylet 7.

As shown in Figures 5 and 6, the braid 85 of the guide wire assembly 8 is inserted into the stylet 7 until the edge of the sleeve 84 abuts against the edge of the stylet 7. The stylet 7 is then inserted into the needle cannula 4. In fact, it must be considered that the braid 85 is extremely flexible and cannot be guided alone inside the needle cannula 4.

Even if not shown in the figures, a biocompatible tubular member having a lower stiffness than the memory-shape metal wire of the tip 80 can be used instead of the braid of metal wires 85. For example, a thin, flexible thread of biocompatible plastic material such as, for example, a polyamide (nylon) thread can be used. In any case, the nylon thread must be less stiff than the tip 80 of the memory-shape metal wire. Specifically, a nylon thread with the same or with a slightly smaller diameter than that of the metal wire of the tip 80 has stiffness characteristics similar to those of the braid of metal wires and can therefore perform the same functions.

As shown in Figure 5, the tip 80 of the guide wire 8 is deformed into a substantially straight shape, inside the needle cannula 5, without exiting from the tip 40 of the needle cannula. The needle cannula 5 containing the stylet 7 and the guide wire 8 is inserted into the patient's body, until the tip 40 of the needle cannula arrives in proximity to the neoplasia to be removed.

At this point, as shown in Figure 6, the needle cannula 4 is retracted and the tip 80 of the guide wire 8 exits from the tip of the needle cannula 4, anchoring itself to the patient's body near the neoplasia. When the tip 80 of the guide wire 8 leaves the needle cannula, it takes on its original curled shape and then anchors itself stably to the patient's body tissue.

If the tip 80 has not been placed in the desired position, it can be inserted into the needle cannula 4 again and then the needle cannula 4 can be re-positioned again in the correct position and retracted, so that the tip 80 is anchored in the correct position.

At this point the screw 53 of the stylet support (Figure 4) is loosened to release the braid 85 therefrom. The needle cannula 4 and the stylet 7 are then extracted from the patient's body, leaving the guide wire 8 in the patient's body, with the tip 80 firmly anchored in proximity to the neoplasia and with the proximal end of the braid 85 protruding on the outside of the patient's body.

In this situation, the tip 80 of the guide wire 8 does not move from its anchoring position, both because its curled shape prevents any movement and because the braid 85 is not very stiff and is very flexible, so it does not transmit to the tip 80 the tensions due to the movement of the part of the user's body around the braid 85.

Furthermore, it must be considered that the low stiffness of the braid 85 or of the nylon thread and its reduced thickness result in a greater comfort for the patient who is obliged to keep the guide wire in place for as long as a few days, before undergoing surgery.

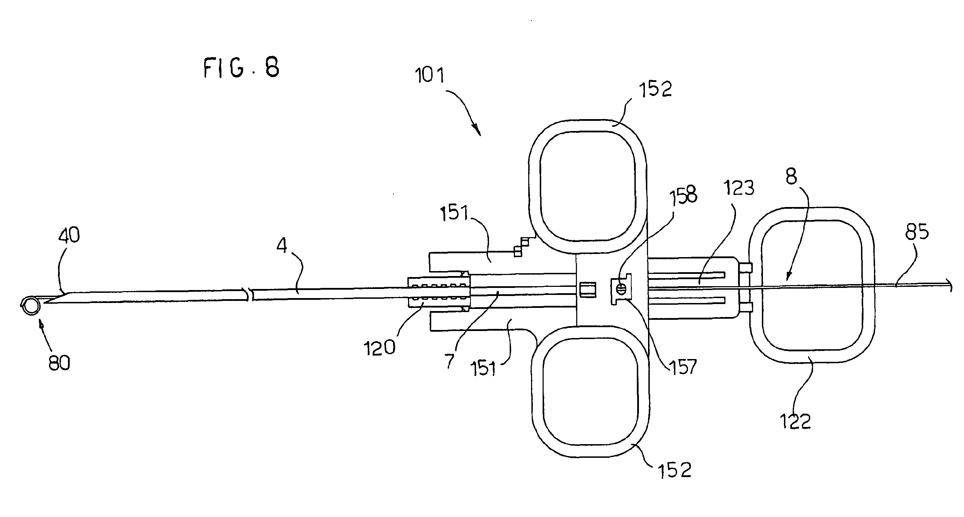

With reference to Figures 7-14 a guide device according to a second embodiment of the invention is described, designated as a whole with reference numeral 101, in which the guide wire assembly 8 is the same as that of the first embodiment and like or corresponding elements to those already described are designated with the same reference numerals and are not described in detail.

The guide device 101 comprises a first support 102 for the needle cannula 4 in the form of a slider provided with an operating ring 122 and a second support 105 for the stylet 7 in the form of a slider provided with two operating rings 152.

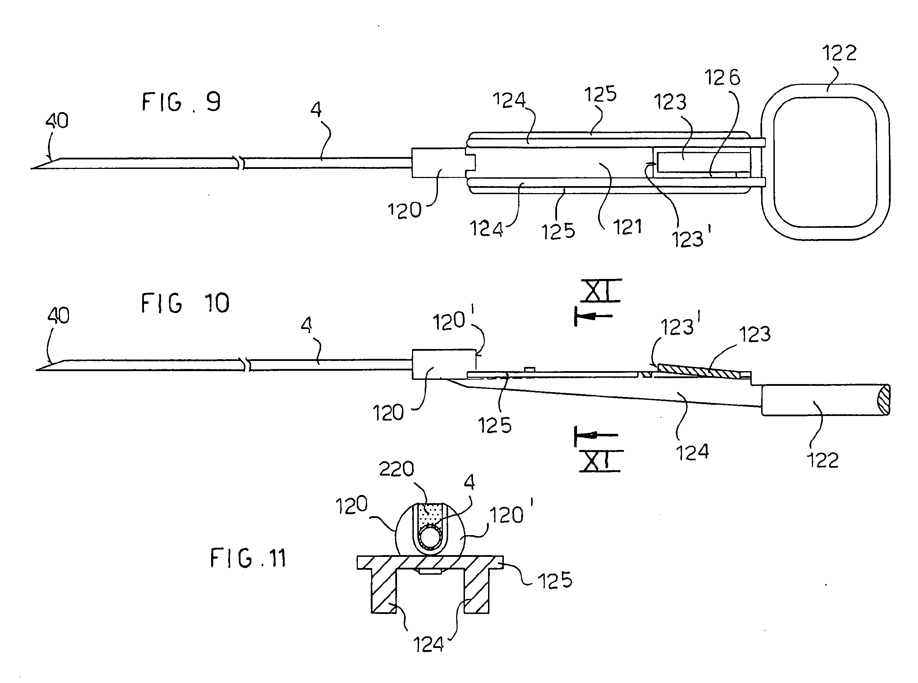

The cannula support 102 has a distal tang 120, substantially U-shaped in cross section (Figure 11), adapted to receive the proximal part of the needle cannula 4 so that the needle cannula 4 protrudes axially therefrom. The needle cannula 4 is secured in the U-shaped seat of the tang 120, by means of the application of an adhesive glue 220 or by means of heat bonding.

The tang 120 is connected to a central body in the form of a rectangular plate 121. The tang 120 protrudes upwards from the body 121, so as to define a rear abutment surface 120'.

A U-shaped cut 126 (Figure 7) which defines a flexible tongue 123 inclined slightly upwardly (Figure 10) so as to present a front abutment surface 123' is made in the plate 121. Two longitudinal ribs 124 which define two side rails 125 are formed on the lower surface of the plate 121.

The support 105 of the stylet 7 has a plate-shaped body 150 in which is embedded the stylet 7 which protrudes forward therefrom. In the proximal end of the plate 150 there is an axial hole 159 communicating with the stylet 7, from which the braid 85 exits.

Two tongues 151 protrude distally from the body 150 on one side and the other with respect to the stylet 7. Two longitudinal ribs 154 connected to retaining ribs 155 parallel to the plane of the plate 150 are formed in the lower surface of the plate 150 and of the tongues 151. In this manner two C-shaped seats 156 (Figure 13) adapted to receive the rails 125 of the cannula support 102 are defined in the lower part of the stylet support 105. The cannula support 102 can thus slide in the beneath the stylet support 105.

A tang 157, provided with a threaded hole 158 that communicates radially with the channel 159 through which the braid 85 of the guide wire assembly 8 passes is provided on the plate 150. A screw (no shown, but similar to the screw 53 of Figure 4) can be screwed into the hole 158 to block the braid 85 in the body 150 of the stylet support.

The guide device 101 can be operated with one hand and is particularly useful in the case of ultrasonography, in which the operator has one hand occupied by the ultrasound probe. In fact, the operator places a thumb in the ring 122 of the cannula support device and the index and middle fingers in the rings 152 of the stylet support device.

Operation of the guide device 101 is described hereunder. Initially the operator retracts the stylet support 105, until the rear surface of the plate 150 of the stylet support 105 abuts against the abutment surface 123' of the flexible tongue 123 of the cannula support 102. In this situation the tip 80 of the guide wire 8 is inside the cannula 4.

The operator then inserts the tip 40 of the cannula body into the patient's body, guiding it, under the guidance of the ultrasound probe, towards the neoplasia that must be removed. When the tip 40 of the cannula 4 is in proximity to the neoplasia, the operator retracts the cannula support 102. As a result, the tip 80 of the guide wire 8 exits from the tip 40 of the cannula 4, anchoring itself firmly to the body tissue, near the neoplasia. If the anchoring position of the tip 80 is not correct, the operator can readily reinsert the tip 80 inside the cannula 4 and re-position it in the correct position.

It should be noted that the stroke of the stylet support 105, with respect to the cannula support 102 is limited by a proximal end-of-stroke represented by the abutment surface 123' of the tongue which abuts against the rear wall of the body 150 of the stylet support 105 and by a distal end-of-stroke represented by the rear edge 120' of the tang 120 of the cannula support 102 which abuts against the front wall of the body 150 of the stylet support 105. The stroke of the stylet support 105 with respect to the cannula support 102 is selected so that when the stylet support 105 is situated at the distal end-of-stroke point, only the tip 80 of the guide wire 8 which must be anchored to the patient's body tissue exits from the tip 40 of the cannula 4 and the connecting sleeve 84 remains protected inside the cannula 4.

Once the correct anchoring position has been found, the operator unscrews the screw that locks the braid 85 to the stylet support 105 and extracts the cannula 4 and the stylet 7 from the patient's body, leaving the guide wire 8 with the tip 80 anchored in the correct position and the braid 85 which protrudes from the patient's body.

In the preferred embodiments of the invention the following have been illustrated:
- a guide wire assembly 8 comprising a tip 80 of a memory-shape metal wire connected, by means of a sleeve 84, to a nylon thread or a braid 85 of metal wires, and
- a stylet 7 adapted to contain the nylon thread or the braid 85 which are not very stiff and are not self-supporting.

However, it must be considered that the invention extends generically to a guide wire 8 in a single piece, without the nylon thread or braid, having a tip 80 in the shape of a curl, which ensures a firm anchoring. In this case, the guide wire 8 has sufficient stiffness to support itself and thus use of the stylet proves superfluous.

Numerous changes and modifications of detail within the reach of a person skilled in the art can be made to the present embodiments without thereby departing from the scope of the invention as set forth in the appended claims.

## Claims

1. A guide device (1; 101) for the localisation of a neoplasia that must be removed during a surgical procedure, comprising:
- a support (2; 102) that supports a cannula (4), hollow on the inside, provided with a percutaneous tip (40) adapted to penetrate the patient's body, and
- a guide wire assembly (8) adapted to be inserted slidably into said cannula (4) and provided with a tip (80) adapted to be anchored to a body tissue inside the patient's body, **wherein** said tip (80) of the guide wire (8) is made from a memory-shape metal wire and has an initial curled shape that forms at least a 360° loop, **characterised in that**
- it further comprises a second support (5, 105) supporting a stylet (7), hollow on the inside and adapted to be inserted slidably in the cannula (4) and **in that**
- said tip (80) of the guide wire (8) is connected to a member (85), with less stiffness than said tip (80), adapted to be disposed slidably inside said stylet (7).

2. A guide device (1; 101) according to claim 1, **characterised in that** said tip (80) of the guide wire (8) forms a 360° loop plus an arc of 30°-60°, preferably 45°.

3. A guide device (1; 101) according to claim 1 or 2, **characterised in that** said tip (80) of the guide wire (8) forms a complete circumference with a diameter of 5 - 10 mm, preferably 8 mm.

4. A guide device (1; 101) according to any one of the preceding claims, **characterised in that** said tip (80) of the guide wire (8) is made from a nickel-titanium alloy wire, with a diameter of 0.3-0.6 mm.

5. A guide device (1; 101) according to **anyone of the preceding claims** , **characterised in that** said tip (80) of the guide wire (8) is connected to said less stiff member (85) by means of a connecting sleeve (84).

6. A guide device (1; 101) according to claim **5, characterised in that** the proximal end of said tip (80) of the guide wire (8) and the distal end of said less stiff member (85) are secured inside said connecting sleeve (84) by crimping (86a, 86b).

7. A guide device (1; 101) according to **anyone** of claims **1** to **6, characterised in that** said member (85) has an outside diameter smaller than the outside diameter of said tip (80).

8. A guide device (1; 101) according to any one of claims **1** to **7, characterised in that** it further comprises screw means (53) that can be operated manually by the operator to block said member (85) inside said support (5, 105) of the stylet (7).

9. A guide device (1; 101) according to any one of claims **1** to **8, characterised in that** said member (85) of the guide wire (8) is a braid of biocompatible stainless steel wires with a diameter of a few microns.

10. A guide device (1; 101) according to any one of claims **1** to **8, characterised in that** said member (85) of the guide wire (8) is a polyamide thread.

11. A guide device (101) according to any one of claims **1** to **10, characterised in that** said cannula support (102) comprises a ring (122) adapted to receive the operator's thumb and said stylet support (105) comprises two rings (152) adapted to receive the operator's index and middle fingers and **in that** said cannula support (102) is mounted slidably beneath said stylet support (105).

12. A guide device (101) according to claim **11, characterised in that** said cannula support (102) has abutment surfaces (123', 120') acting as end-of-stroke for said stylet support (105), so that the stylet support (105) can slide from a retracted position in which the tip (80) of the guide wire (8) is inside the cannula (4) to an advanced position in which the tip (80) of the guide wire (8) is outside the tip (40) of the cannula (4).

## Patentansprüche

1. Führungsvorrichtung (1; 101) zur Lokalisierung einer Neoplasie, die während eines chirurgischen Verfahrens entfernt werden muss, umfassend:
- einen Träger (2; 102), der ein Kanüle (4) trägt, die an der Innenseite hohl ist und mit einer perkutanen Spitze (40) bereitgestellt ist, die dazu ausgebildet ist, in den Körper des Patienten einzudringen, und
- eine Führungsdrahtanordnung (8), die dazu ausgebildet ist, gleitend in die Kanüle (4) eingesetzt zu werden, und mit einer Spitze (80) versehen ist, die dazu ausgebildet ist, an einem Körpergewebe im Inneren des Körpers des Patienten verankert zu werden, wobei die Spitze (80) des Führungsdrahtes (8) aus einem Formgedächtnismetalldraht gebildet ist und eine anfänglich gekringelte Form aufweist, die mindestens eine 360° Schlinge bildet, **dadurch gekennzeichnet, dass**
- sie des Weiteren einen zweiten Träger (5, 105) umfasst, der ein Stilett (7) trägt, das an der Innenseite hohl ist und dazu ausgebildet ist, in die Kanüle (4) eingesetzt zu werden, und dass
- die Spitze (80) des Führungsdrahtes (8) mit einem Element (85) mit weniger Steifigkeit als die Spitze (80) verbunden ist, das dazu ausgebildet ist, gleitend im Inneren des Stiletts (7) angeordnet zu werden.

2. Führungsvorrichtung (1; 101) nach Anspruch 1, **dadurch gekennzeichnet, dass** die Spitze (80) des Führungsdrahtes (8) eine 360° Schlinge plus einem Bogen von 30°-60°, vorzugsweise 45° bildet.

3. Führungsvorrichtung (1; 101) nach Anspruch 1 oder 2, **dadurch gekennzeichnet, dass** die Spitze (80) des Führungsdrahtes (8) einen vollständigen Kreisumfang mit einem Durchmesser von 5 bis 10 mm, vorzugsweise 8 mm bildet.

4. Führungsvorrichtung (1; 101) nach einem der vorangehenden Ansprüche, **dadurch gekennzeichnet, dass** die Spitze (80) des Führungsdrahtes (8) aus einem Nickel-Titan-Legierungsdraht mit einem Durchmesser von 0,3 bis 0,6 mm besteht.

5. Führungsvorrichtung (1; 101) nach einem der vorangehenden Ansprüche, **dadurch gekennzeichnet, dass** die Spitze (80) des Führungsdrahtes (8) mit dem weniger steifen Element (85) mit Hilfe einer Verbindungshülse (84) verbunden ist.

6. Führungsvorrichtung (1; 101) nach Anspruch 5, **dadurch gekennzeichnet, dass** das proximale Ende der Spitze (80) des Führungsdrahtes (8) und das distale Ende des weniger steifen Elements (85) im Inneren der Verbindungshülse (84) durch Bördeln (86a, 86b) befestigt sind.

7. Führungsvorrichtung (1; 101) nach einem der Ansprüche 1 bis 6, **dadurch gekennzeichnet, dass** das Element (85) einen Außendurchmesser aufweist, der kleiner als der Außendurchmesser der Spitze (80) ist.

8. Führungsvorrichtung (1; 101) nach einem der Ansprüche 1 bis 7, **dadurch gekennzeichnet, dass** sie des Weiteren Schraubenmittel (53) umfasst, die von dem Bediener manuell betätigt werden können, um das Element (85) im Inneren des Trägers (5, 105) des Stiletts (7) zu blockieren.

9. Führungsvorrichtung (1; 101) nach einem der Ansprüche 1 bis 8, **dadurch gekennzeichnet, dass** das Element (85) des Führungsdrahtes (8) ein Geflecht aus biokompatiblen Drähten aus rostfreiem Stahl mit einem Durchmesser von wenigen Mikron ist.

10. Führungsvorrichtung (1; 101) nach einem der Ansprüche 1 bis 8, **dadurch gekennzeichnet, dass** das Element (85) des Führungsdrahtes (8) ein Polyamidfaden ist.

11. Führungsvorrichtung (1; 101) nach einem der Ansprüche 1 bis 10, **dadurch gekennzeichnet, dass** der Kanülenträger (102) einen Ring (122) umfasst, der zur Aufnahme des Daumens des Bedieners ausgebildet ist, und der Stilettträger (105) zwei Ringe (152) umfasst, die zur Aufnahme des Zeigefingers und Mittelfingers des Bedieners ausgebildet sind, und dass der Kanülenträger (102) gleitend unterhalb des Stilettträgers (105) montiert ist.

12. Führungsvorrichtung (1; 101) nach Anspruch 11, **dadurch gekennzeichnet, dass** der Kanülenträger (102) Auflageflächen (123', 120') hat, die als Hubbegrenzung für den Stilettträger (105) dienen, so dass der Stilettträger (105) aus einer zurückgezogenen Position, in der die Spitze (80) des Führungsdrahtes (8) im Inneren der Kanüle (4) liegt, zu einer vorgeschobenen Position, in der sich die Spitze (80) des Führungsdrahtes (8) außerhalb der Spitze (40) der Kanüle (4) befindet, gleiten kann.

## Revendications

1. Dispositif de guidage (1 ; 101) pour la localisation d'une néoplasie à extraire lors d'une intervention chirurgicale, comprenant :
- un support (2; 102) qui supporte une canule (4) creuse à l'intérieur et dotée d'une pointe percutanée (40) apte à pénétrer dans le corps du patient et
- un assemblage de fil de guidage (8) apte à être inséré en glissant dans ladite canule (4) et doté d'une pointe (80) apte à être ancrée dans un tissu corporel dans le corps du patient, ladite pointe (80) du fil de guidage (8) étant faite d'un fil métallique à mémoire de forme et ayant une forme initiale ondulée qui forme au moins une boucle de 360°, **caractérisé en ce que**
- il comprend en outre un second support (5, 105) supportant un stylet (7) creux à l'intérieur et apte à être inséré en glissant dans la canule (4) et **en ce que**
- ladite pointe (80) du fil de guidage (8) est connectée à un élément (85) moins rigide que ladite pointe (80) et apte à être disposé en glissant à l'intérieur dudit stylet (7).

2. Dispositif de guidage (1 ; 101) selon la revendication 1, **caractérisé en ce que** ladite pointe (80) du fil de guidage (8) forme une boucle à 360° plus un arc de 30 à 60°, de préférence 45°.

3. Dispositif de guidage (1 ; 101) selon la revendication 1 ou 2, **caractérisé en ce que** ladite pointe (80) du fil de guidage (8) forme une circonférence complète ayant un diamètre de 5 à 10 mm, de préférence 8 mm.

4. Dispositif de guidage (1; 101) selon l'une quelconque des revendications précédentes, **caractérisé en ce que** ladite pointe (80) du fil de guidage (8) est faite d'un fil en alliage de nickel/titane ayant un diamètre de 0,3 à 0,6 mm.

5. Dispositif de guidage (1; 101) selon l'une quelconque des revendications précédentes, **caractérisé en ce que** ladite pointe (80) du fil de guidage (8) est connectée audit élément moins rigide (85) au moyen d'un manchon connecteur (84).

6. Dispositif de guidage (1 ; 101) selon la revendication 5, **caractérisé en ce que** ladite pointe (80) du fil de guidage (8) et l'extrémité distale dudit élément moins rigide (85) sont bloquées à l'intérieur dudit manchon connecteur (84) par sertissage (86a, 86b).

7. Dispositif de guidage (1 ; 101) selon l'une quelconque des revendications 1 à 6, **caractérisé en ce que** ledit élément (85) a un diamètre extérieur inférieur au diamètre extérieur de ladite pointe (80).

8. Dispositif de guidage (1 ; 101) selon l'une quelconque des revendications 1 à 7, **caractérisé en ce qu'**il comprend en outre un moyen de vissage (53) qui peut être actionné manuellement par l'opérateur pour bloquer ledit élément (85) à l'intérieur dudit support (5, 105) du stylet (7).

9. Dispositif de guidage (1 ; 101) selon l'une quelconque des revendications 1 à 8, **caractérisé en ce que** ledit élément (85) du fil de guidage (8) est une tresse de fils en acier inox biocompatible ayant un diamètre de quelques microns.

10. Dispositif de guidage (1 ; 101) selon l'une quelconque des revendications 1 à 8, **caractérisé en ce que** ledit élément (85) du fil de guidage (8) est un fil de polyamide.

11. Dispositif de guidage (1 ; 101) selon l'une quelconque des revendications 1 à 10, **caractérisé en ce que** ledit support de canule (102) comporte une bague (122) apte à recevoir le pouce de l'opérateur et que ledit support de stylet (105) comporte deux bagues (152) aptes à recevoir l'index et les doigts du milieu de l'opérateur et **en ce que** ledit support de canule (102) est monté de manière à pouvoir glisser sous ledit support de stylet (105).

12. Dispositif de guidage (1 ; 101) selon la revendication 11, **caractérisé en ce que** ledit support de canule (102) a des surfaces de butée (123', 120') faisant office de butée de fin de course pour ledit support de stylet (105), de sorte que le support de stylet (105) peut glisser depuis une position rétractée dans laquelle la pointe (80) du fil de guidage (8) est à l'intérieur de la canule (4) vers une position avancée dans laquelle la pointe (80) du fil de guidage (8) est située à l'extérieur de la pointe (40) de la canule (4).
